# EUROPÄISCHE PATENTSCHRIFT

(11) **EP 1 986 590 B1**
(45) Veröffentlichungstag und Bekanntmachung des Hinweises auf die Patenterteilung: **16.11.2011**
(21) Anmeldenummer: 07703052.6
(22) Anmeldetag: 26.01.2007
(51) Int. Cl.: A61K 8/02, A61K 8/34, A61K 8/36, A61K 8/37, A61K 8/39, A61Q 5/06, A61Q 5/10, C11D 1/04, C11D 1/72, C11D 1/83, C11D 3/20

(54) **RHEOPEXE GELE ALS TRÄGERMEDIUM**
RHEOPEXY GELS AS VEHICLES
GELS RHEOPECTIQUES UTILISES COMME MILIEU DE SUPPORT

(30) Priorität: 07.02.2006 DE 102006005768
(43) Veröffentlichungstag der Anmeldung: 05.11.2008
(73) Patentinhaber: Henkel AG & Co. KGaA, 40589 Düsseldorf (DE)
(72) Erfinder: REICHERT, Anja, 40629 Düsseldorf (DE); GOUTSIS, Konstantin, 41812 Erkelenz (DE)
(86) Internationale Anmeldenummer: PCT/EP2007/000663
(87) Internationale Veröffentlichungsnummer: WO 2007/093271

(56) Entgegenhaltungen:
- WO-A-97/28785
- WO-A-2006/015650
- DE-A1- 10 129 034
- DE-A1- 19 754 281
- US-A1- 2003 012 759

## Beschreibung

Gegenstand der Erfindung sind Zusammensetzungen in Gel-Form, die ein rheopexes Viskositätsverhalten aufweisen, sowie deren Verwendung als kosmetische Trägermedien, insbesondere für oxidative Färbemittel.

Für das Färben von Keratinfasern, insbesondere von Haaren, Pelzen und Fellen spielen die sogenannten Oxidationsfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Eine bestimmte Entwicklersubstanz kann durch Kombination mit unterschiedlichen Kupplern auch sehr unterschiedliche Farbnuancen bilden. Trotzdem gelingt es oft nicht, mit Hilfe einer einzigen Entwicklersubstanz zu einer Vielzahl natürlicher Farbnuancen zu kommen. In der Praxis ist daher meist eine Kombination verschiedener Entwicklerkomponenten und Kupplerkomponenten erforderlich, um eine einzige, natürlich wirkende Färbung zu erhalten.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Bei ihrer Anwendung in Haarfärbemitteln sollen sie bereits bei Temperaturen unterhalb 40°C ein gutes Aufziehvermögen auf menschlichem Haar besitzen, wobei keine merklichen Unterschiede zwischen strapaziertem und frisch nachgewachsenem Haar bestehen dürfen (Egalisiervermögen). Sie sollen beständig sein gegen Licht, Wärme und den Einfluß von Haarshampoos und chemischen Reduktionsmitteln, z. B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Oxidationshaarfärbemittel in Gel-Form werden vom Verbraucher als besonders ästhetische Anbietungsform empfunden. Der Begriff "Gel" wird in "Faraday discussions of the chemical society, No. 57, 1974 (Gels and Gelling Processes), P.J. Flory, Seite 7-18", näher charakterisiert. Demnach werden Gele in 4 Untergruppen eingeteilt:
1. Gele mit lamellaren Ordnungsstrukturen einschließlich Gel-Mesophasen, z.B. Tensidgele, insbesondere Seifengele.
2. Gele, die aus polymeren, dreidimensionalen, kovalent gebundenen Netzwerken, die ohne Zerstörung der Kovalenzbindungen in Lösungsmitteln unlöslich sind, aufgebaut sind.
3. Gele aus polymeren Netzwerken mit lokalen Ordnungsstrukturen, die durch physikalische Kräfte zusammengehalten werden, z.B. Gelatine, Homo- und Copolymere von Acryl- und Methacrylsäure, Alginate, Carrageenan.
4. Gele gebildet aus feinverteilten Bestandteilen, z.B. Niederschlägen, die i.a. eine große geometrische Anisotropie aufweisen, z.B. V₂O₅-Gele oder Gele, die sich durch Aggregation von Proteinen bilden.

Gele im Sinne der nachfolgend beschriebenen Erfindung sind solche, die unter Punkt 1. und Punkt 3. der obigen Auflistung fallen. Als "Gel" im Sinne der vorliegenden Erfindung sind auch Transparentgele wie sie z.B. in den US-Patentschriften US 3 101 300 und US 3 101 301 beschrieben werden, zu verstehen. Diese Transparentgele werden als Mikroemulsionsgele bezeichnet. Eine allgemeingültige Definition für Mikroemulsionsgele ist in "Happi: Household Pers. Prod. Ind. 30 (1993), Nr. 2, p. 58-64" angegeben.

Im Vergleich zu Oxidationshaarfärbemitteln in Creme-Form bieten Färbemittel in Gel-Form einige anwendungstechnische Vorteile. So ist die Herstellung von Cremes (i.a. O/W-Emulsionen) generell problematischer und aufwendiger; Cremes dicken oft nach, was für 2-Komponenten-Verpackungen besonders nachteilig ist. Die in den Rahmen der Erfindung fallenden Gele hingegen behalten ihre einmal eingestellte Viskosität über einen längeren Zeitraum bei und lassen sich leichter auf dem Kopfhaar verteilen.

Im Stand der Technik finden sich zwar Beispiele für Oxidationshaarfärbemittel in Gel-Form, allerdings sind diese Gele verbesserungswürdig bezüglich ihrer Anwendungsparameter.

So werden in den deutschen Offenlegungsschriften DE 33 02 534 (Beispiel 41) und DE 34 21 694 (Beispiele 27, 41, 42) Oxidationshaarfärbemittel in Gel-Form offenbart.

Die deutsche Offenlegungsschrift DE 40 22 847 beschreibt gelförmige Oxidationshaarfärbemittel.

In "Hair dyes, J.C. Johnson, Noyes Data Corporation 1973, Seiten 313 - 330" werden Gel-Oxidationshaarfärbemittel offenbart.

In der europäischen Patentschrift EP-B1-1 035 825 werden fließfähige gelförmige Träger für oxidative Färbemittel offenbart. Diese Träger weisen kein rheopexes Verhalten auf und enthalten zwingend niedermolekulare Alkohole wie Isopropanol.

Die Patentanmeldung DE 102004039181.5 betrifft gelförmige Zusammensetzungen, die stabil mit einem hohen Gehalt an Elektrolyten formuliert werden können. Diese Gele besitzen kein rheopexes Verhalten.

Aufgabe der vorliegenden Erfindung ist es daher, eine leicht handhabbare gelförmige Zusammensetzung in lagerstabiler Form bereitzustellen. Die Gelformulierung sollte ohne Einfluß von Scherkräften fließfähig sein. Wenn die gelförmige Formulierung Scherkräften ausgesetzt wird, sollte sich das Gel verdicken. Auf die Verwendung von niedermolekularen Monoalkoholen zur Einstellung der Fließfähigkeit sollte weitgehend verzichtet werden.

Es wurde nun überraschenderweise gefunden, dass die erfindungsgemäße Kombination von Komponenten ein lagerstabiles Gel mit der oben beschriebenen Rheopexie liefert. Die Zusammensetzungen besitzen hervorragende Eigenschaften in der Anwendung, insbesondere am Haar. Sie lassen sich leicht aus einem Behältnis ausgiessen und bilden dabei bereits eine erhöhte Viskosität aus. Die Gele, ob im fließfähigen oder verdickten Zustand, sind hervorragend mit wässrigen Systemen mischbar. Wenn die Zusammensetzungen als Trägermedium für oxidative Haarfärbemittel Anwendung finden, erlauben sie haltbare und intensive oxidative Färbungen.

Gegenstand der Erfindung ist eine gelförmige Zusammensetzung, insbesondere als kosmetischer Träger für Oxidationsfarbstoffvorprodukte, enthaltend
- Wasser,
- 1,0 bis 15 Gew.-% eines gesättigten oder ungesättigten, linearen oder verzweigten Alkohols mit 8 bis 36 C-Atomen,
- 0,1 bis 15 Gew.-% einer flüssigen Fettsäuren mit 16 bis 22 C-Atomen in Form einer wasserlöslichen Seife,
- 0,5 bis 10 Gew.-% eines Anlagerungsproduktes von 1 bis 5 Mol Ethylenoxid an einen linearen Fettalkohol mit 8 bis 22 C-Atomen
und
- 0,5 bis 10 Gew.-% mindestens eines Esteröls ausgewählt aus
   (i) C₁- bis C₁₀-Alkyl-(C₈- bis C₃₀-)alkanoaten, deren Kohlenwasserstoffreste linear oder verzweigt, gesättigt oder ungesättigt sein können und/oder
   (ii) C₈- bis C₃₀-Alkyl-(C₁- bis C₁₀-)alkanoaten, deren Kohlenwasserstoffreste linear oder verzweigt, gesättigt oder ungesättigt sein können.

Die erfindungsgemäßen Zusammensetzungen enthalten bevorzugt mindestens 30 Gew.-%, besonders bevorzugt mindestens 60 Gew.-% Wasser, bezogen auf das Gewicht der gesamten Zusammensetzung.

Bei den erfindungsgemäß einzusetzenden gesättigten oder ungesättigten, linearen oder verzweigten Alkoholen mit 8 bis 36 C-Atomen handelt es sich vorzugsweise um Fettalkohole und/oder Guerbetalkohole.

Unter Fettalkoholen sind primäre aliphatische Alkohole der Formel (I) zu verstehen,

R¹OH (I)

in der R¹ für einen aliphatischen, linearen oder verzweigten Kohlenwasserstoffrest mit 8 bis 36 Kohlenstoffatomen, insbesondere 8 bis 22 Kohlenstoffatomen, der jeweils gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind Fettalkohole mit 12 bis 18 Kohlenstoffatomen wie beispielsweise Fettalkoholmischungen des Kokos- oder Palm-, Palmkern- oder Talgfettalkohols, insbesondere des Kokos- und/oder Talgfettalkohols. Besonders bevorzugte Fettalkoholmischungen enthalten Fettalkohole mit 12-, 16- und 18-Kohlenstoffatomen.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Die zur Seifenbildung geeigneten Fettsäuren können gesättigt oder ungesättigt, linear oder verzweigt sein, sie sind bevorzugt ungesättigt oder verzweigt. Ferner sind erfindungsgemäß flüssige ungesättigte C₁₆-C₂₂-Fettsäuren bevorzugt, wie Palmitoleinsäure, Ölsäure, Elaidinsäure, Myristinsäure, Petroselinsäure, Petroselaidinsäure, Gadoleinsäure, Erucasäure, Brassidinsäure sowie Gemische dieser Fettsäuren untereinander und gegebenenfalls mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyldecansäure, Isostearinsäure und die 2-Octyldodecansäure.

Die flüssigen Fettsäuren sind im Sinne der Erfindung bevorzugt bei einer Temperatur von 20°C flüssig.

Zur Überführung der besagten Fettsäuren in wasserlösliche Seifen eignen sich Alkalihydroxide und Alkalicarbonate, Ammoniak, Mono-, Di- und Trialkanolamine mit 2 bis 4 C-Atomen in der Alkanolgruppe sowie alkalische Aminosäuren wie beispielsweise Arginin, Ornithin, Lysin und/ oder Histidin.

Als Anlagerungsprodukte von 1 bis 4 bzw. 15 bis 100 Mol Ethylenoxid an einen linearen Fettalkohol mit 12 bis 22 C-Atomen eignen sich alle nach den bekannten technischen Oxethylierungsverfahren erhältlichen Addukte. Bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Die in den erfindungsgemäßen Zusammensetzungen enthaltenen Esteröle sind bevorzugt bei einer Temperatur von 6°C flüssig.

Die erfindungsgemäß verwendeten Esteröle besitzen bevorzugt eine Struktur gemäß Formel (II) 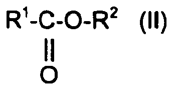 worin
einer der Reste R¹ oder R² für einen linearen oder verzweigten, gesättigten oder ungesättigten (C₂- bis C₁₀)-Kohlenwasserstoffrest steht und der jeweils andere Rest einen linearen oder verzweigten, gesättigten oder ungesättigten (C₈- bis C₂₂)-Kohlenwasserstoffrest bedeutet. Die Kohlenwasserstoffreste R¹ und R² der Formel (II) tragen wie oben definiert keinen weiteren Substituenten.

Erfindungsgemäß besonders bevorzugte Esteröle sind ausgewählt aus mindestens einem Vertreter der Gruppe, die gebildet wird, aus Isopropylmyristat (Rilanit^{®} IPM), Isononansäure-C₁₆₋₁₈-alkylester (Cetiol^{®} SN), 2-Ethylhexylpalmitat (Cegesoft^{®} 24), Stearinsäure-2-ethylhexylester (Cetiol^{®} 868), Cetyloleat, Kokosfettalkohol-caprinat/- caprylat (Cetiol^{®} LC), n-Butylstearat, Oleylerucat (Cetiol^{®} J 600), Isopropylpalmitat (Rilanit^{®} IPP), Laurinsäurehexylester (Cetiol^{®} A), Cetearyl Isononanoate (Cetiol^{®} SN) und Ölsäuredecylester (Cetiol^{®} V).

Besonders bevorzugte erfindungsgemäße Esteröle besitzen mindestes einen verzweigten Kohlenwasserstoffrest.

Die erfindungsgemäße Zusammensetzung kann, wenn Elektrolyte in das Gel eingearbeitet werden sollen, zusätzlich zur Stabilisierung 0,5 bis 10 Gew.-% eines Anlagerungsproduktes von 15 bis 100 Einheiten Ethylenoxid pro Molekül eines linearen Fettalkohols mit 8 bis 22 C-Atomen enthalten. Es ist erfindungsgemäß bevorzugt, Anlagerungsprodukte von 15 bis 100 Mol Ethylenoxid, insbesondere von 15 bis 50 Mol Ethylenoxid, an einen linearen Fettalkohol mit 8 bis 22 Kohlenstoffatomen in den gelförmigen Zusammensetzungen zu verwenden. Es handelt sich dabei ganz besonders bevorzugt um Ceteareth-15 oder Ceteareth-50, welche als Eumulgin^{®} CS 15 bzw. Eumulgin^{®} CS 50 von der Firma Cognis Deutschland GmbH vermarktet werden.

Es ist möglich, zur Einstellung der Viskosität der gelförmigen Zusammensetzung zusätzlich mindestens einen niedermolekularen, wasserlöslichen Monoalkohol, beispielsweise in einer Menge von 0,5 bis 10 Gew.-% (bezogen auf das Gewicht der gesamten Zusammensetzung), zuzusetzen. Unter niedermolekularen Monoalkoholen sind im Sinne der vorliegenden Erfindung bei 20°C mit Wasser mischbare Alkohole mit 1 bis 5 C-Atomen zu verstehen. Diese Monoalkohole tragen nur eine Hydroxygruppe. Vorzugsweise handelt es sich um Ethanol, Propanol und/ oder Isopropanol. Es ist jedoch erfindungsgemäß bevorzugt, die Zusammensetzungen frei von niedermolekularen, wasserlöslichen Monoalkoholen zu formulieren.

Weiterhin können die erfindungsgemäßen Zusammensetzungen zusätzlich mindestens ein Polyol enthalten. Polyole tragen im Sinne der Erfindung mindestens zwei Hydroxygruppen, bevorzugt zwei bis vier Hydroxygruppen. Als Polyole sind insbesondere solche mit 2 bis 6 C-Atomen bevorzugt. Es eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist besonders bevorzugt.

Als wasserlösliche synthetische Tenside eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziierte ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und α-Olefinsulfonate mit 12 bis 18 C-Atomen.

Geeignete nichtionogene Tenside sind z.B. die Anlagerungsprodukte von 5 bis 14 Mol Ethylenoxid an lineare Fettalkohole mit 12 bis 18 C-Atomen, die Anlagerungsprodukte von 3 bis 70 Mol an Fettsäuren mit 12 bis 18 C-Atomen sowie an Fettsäuremonoglyceride und an Fettsäure-sorbitanmonoester. Bevorzugt geeignete nichtionogene Tenside sind die Fettalkylaminoxide und insbesondere die Fettalkylglykoside, vorzugsweise Fettalkylglucoside. Die Fettalkylgruppe kann in den genannten Produkten 12 bis 18 C-Atome aufweisen.

Im Sinne der Erfindung bevorzugt einsetzbare zwitterionische Tenside sind N-Fettalkyldimethylammoniumglycinat oder N-Fettacylaminopropyldimethylglycinat.

Weiterhin bevorzugt werden Kationtenside wie wie quartäre Ammoniumverbindungen (QAV) insbesondere quaternierte Trialkylammoniumverbindungen mit Alkylresten einer Kettenlänge von C₈ bis C₂₂.

Zusätzlich können die erfindungsgemäßen Mittel Anlagerungsprodukte von 1 bis 4 Mol Ethylenoxid an ein Fettalkylamin enthalten. Als Anlagerungsprodukte von 1 bis 4 Mol Ethylenoxid an ein lineares Fettalkylamin mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein C₁₂-C₁₈-Kokosalkylamin.

Geeignete Verbindungen der Formel III

R⁵-(OC₂H₄)ₓ-A-(C₂H₄O)_{y}-R⁶ (III)

in welcher A ein Sauerstoffatom ist, sind Dialkylether mit 12 bis 18 C-Atomen in den Alkylgruppen. Solche Produkte sind literaturbekannt. Noch besser geeignet sind die Produkte der Formel III, in welchen x oder y oder beide = 1 sind. Solche Dialkyloxethylether lassen sich durch literaturbekannte Veretherungsverfahren aus Fettalkoholen und Fettalkyloxyethanolen herstellen. Die Produkte der Formel III, in welchen A eine Gruppe 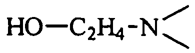 ist, lassen sich z.B. aus Triethanolamin durch O-Alkylierung mit 2 Mol eines Schwefelsäurehalbestersalzes eines C₁₂-C₂₂-Fettalkohols nach dem in DE 35 04 242 beschriebenen Verfahren zur Herstellung von Etheraminen gewinnen.

Besonders bevorzugte Verbindungen der Formel II sind z.B. Dicetylstearylether, Dicetylstearyldioxyethylether und N,N-Bis-(2-cetyl-/stearyl-oxyethyl)-aminoethanol.

Während mit Anlagerungsprodukten von 1 bis 5 Mol Ethylenoxid an einen linearen Fettalkohol die erforderliche Verdickung meist allein durch diese Komponente erreicht wird, kann es bei Einsatz der Anlagerungsprodukte von 1 bis 4 Mol Ethylenoxid an ein lineares Fettalkylamin vorteilhaft sein, diese in Kombination mit 1 bis 10 Gew.-% einer Verbindung der Formel III zu verwenden.

Besonders bevorzugt im Sinne der vorliegenden Erfindung sind amphotere Tenside wie beispielsweise N-Fettalkyl-dimethyl-glycin, N-Fettalkylaminopropionsäure und Tenside der Formel (Amph-1) (*vide infra*).

Als besonders bevorzugt verwendbares amphoteres Tensid eignet sich mindestens eine Verbindung mit der nachstehenden Formel (Amph-1) und/oder dessen physiologisch verträgliche Salze, 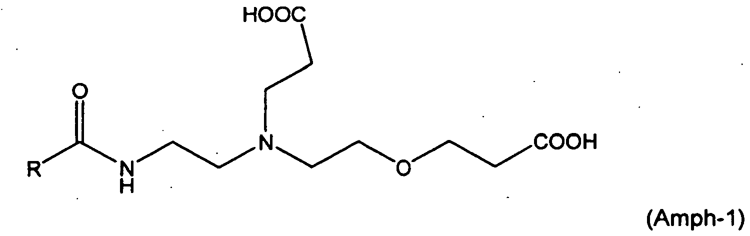 worin R eine lineare oder verzweigte, gesättigte oder ungesättigte (C₁₀ - C₂₂)-Alkylgruppe bedeutet.

In einer bevorzugten Ausführungsform steht der Rest R gemäß Formel (Amph-1) für eine lineare (C₁₂ bis C₁₈)-Alkylgruppe. Besonders bevorzugte Verbindungen der Formel (Amph-1) werden unter der INCI-Bezeichnung Disodium Cocoamphodipropionate mit den Handelsnamen Miranol^{®} C2M SF conc. (Rhodia), Amphoterge^{®} K-2 (Lonza) und Monateric^{®} CEM-38 (Unichema) vermarktet. Dabei leitet sich die Gruppe R-CO- gemäß Formel (Amph-1) von Fettsäuren des Kokosnußöls ab.

Die Verbindungen der Formel (Amph-1) sind in der erfindungsgemäßen Zusammensetzung bevorzugt in einer Menge von 0,05 bis 15 Gew.-%, besonders bevorzugt von 0,1 bis 10 Gew.-%, jeweils bezogen auf das Gewicht der Zusammensetzung, enthalten.

In einer weiteren bevorzugten Ausführungsform enthält die erfindungsgemäße Zusammensetzung zusätzlich mindestens eine Entwicklerkomponente und/oder Kupplerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (Ent1) 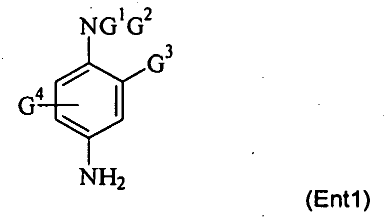 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, C₁-bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (Ent2) entsprechen, sowie ihre physiologisch verträglichen Salze: 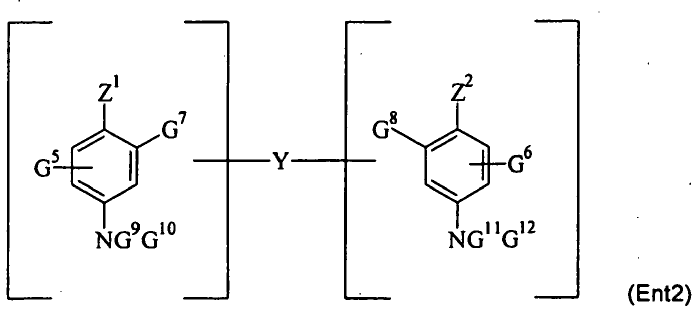 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3) 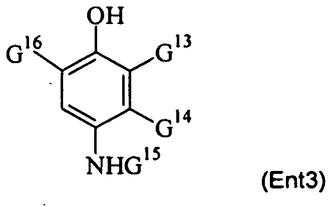 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (Ent3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Bevorzugte Pyrazolopyrimidin-Derivate sind insbesondere die Derivate des Pyrazolo[1,5-a]opyrimidin der folgenden Formel (Ent4) und dessen tautomeren Formen, sofern ein tautomeres Gleichgewicht besteht: 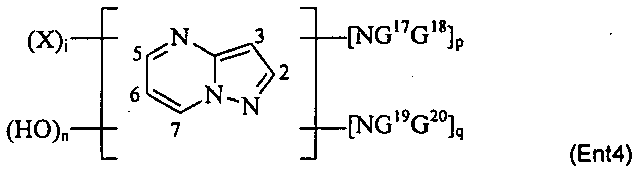 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (Ent4) verwendeten Substituenten sind erfindungsgemäß analog zu den obigen Ausführungen definiert.

Wenn das Pyrazolo[1,5-a]pyrimidin der obenstehenden Formel (Ent4) eine Hydroxygruppe an einer der Positionen 2, 5 oder 7 des Ringsystems enthält, besteht ein tautomeres Gleichgewicht, das zum Beispiel im folgenden Schema dargestellt wird:

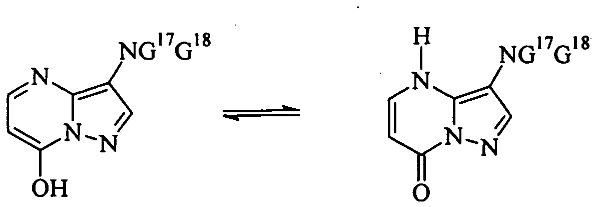

Unter den Pyrazolo[1,5-a]pyrimidinen der obenstehenden Formel (Ent4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]opyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (Ent4) können wie in der Literatur beschrieben durch Zyklisierung ausgehend von einem Aminopyrazol oder von Hydrazin hergestellt werden.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins der Formel (DHIa), 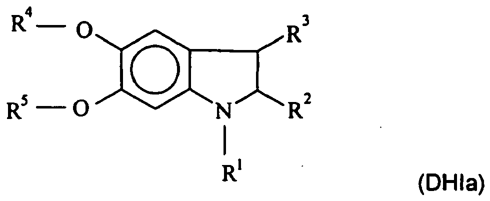 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (DHIb), 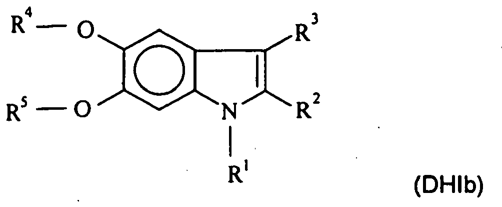 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Wenn das erfindungsgemäße Mittel mindestens eine Kupplerkomponente enthält, werden als erfindungsgemäß bevorzugte Kupplerkomponenten verwendet:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol, o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, . Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Bevorzugte direktziehende Farbstoffe, die in den erfindungsgemäßen Mitteln Verwendung finden, sind Nitrophenylendiamine, Nitroaminophenole, Azofarbstoffe, Anthrachinon oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

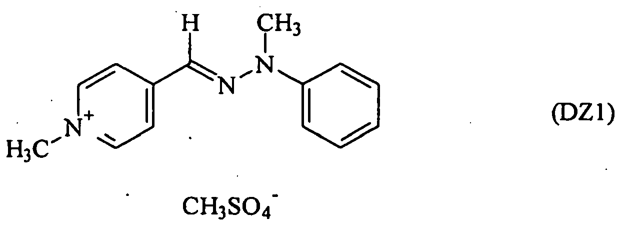

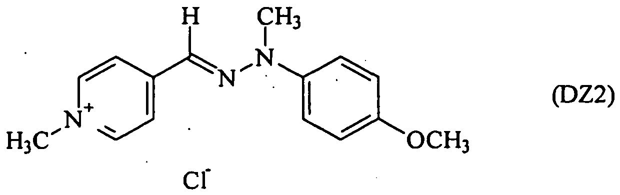

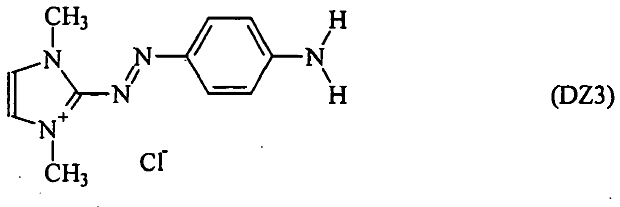

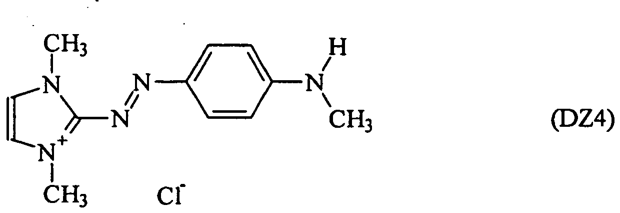

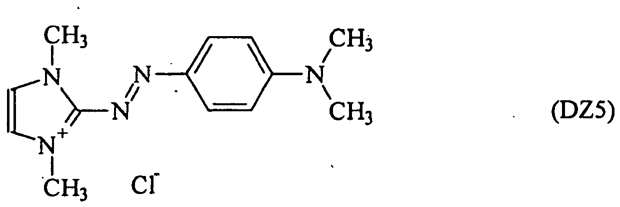

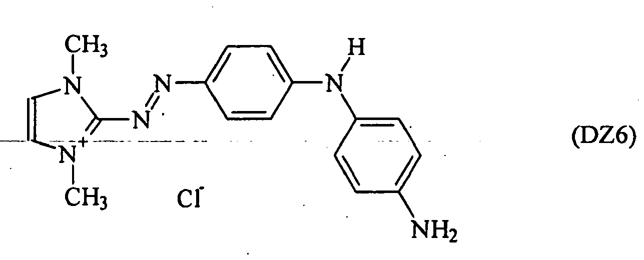

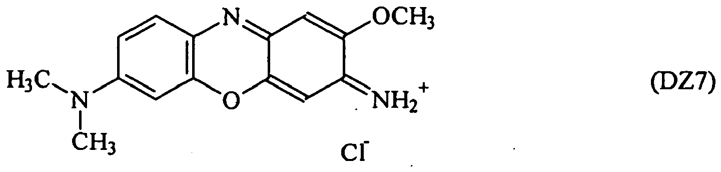

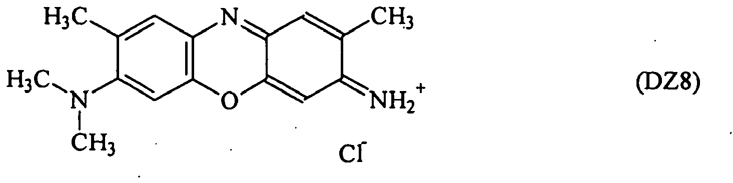

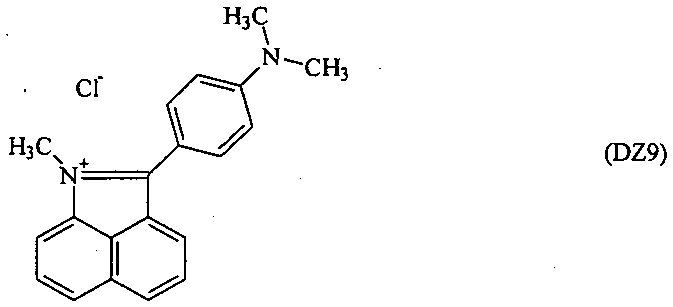

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Zusammensetzungen gemäß dieser Ausführungsform enthalten die direktziehenden Farbstoffe bevorzugt in einer Menge von 0,01 bis 20 Gew.-%, bezo- . gen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Der erfindungsgemäßen Zusammensetzung können Oxidationsfarbstoffvorprodukte bzw. Direktfarbstoffe unmittelbar vor der Anwendung zugemischt werden. Es ist bevorzugt, wenn die erfindungsgemäße Zusammensetzung derart konfektioniert ist, dass zumindest Oxidationsfarbstoffvorprodukte im Gemisch mit der erfindungsgemäßen Zusammensetzung in einem Container aufbewahrt werden.

Die Oxidationsfarbstoffvorprodukte, insbesondere p-Toluylendiamin, werden in den erfindungsgemäßen Zusammensetzungen bevorzugt in Form ihrer physiologisch verträglichen Salze eingesetzt. Diese werden durch Umsetzung des Oxidationsfarbstoffvorprodukts mit einer anorganischen oder organischen Säure gebildet.

Bezüglich der in den erfindungsgemäßen Zusammensetzungen einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als zusätzlicher Verdicker können erfindungsgemäß Xanthan-Gum, Agar-Agar, lineare und vernetzte Polyacrylate, nichtionogene und anionische Cellulosederivate in den erfindungsgemäßen Zusammensetzungen enthalten sein.

Weiterhin können haarkosmetische Hilfsstoffe enthalten sein, insbesondere Bisabolol, Pflanzenextrakte, Vitamine wie vorzugsweise Niacinamid, Tocopherol, Vitamin A, Biotin und Vitamin D.

Wenn die erfindungsgemäße Zusammensetzung Oxidationsfarbstoffvorprodukte enthält, kann die eigentliche oxidative Färbung der Fasern grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Es ist erfindungsgemäß bevorzugt, wenn die gelförmigen Zusammensetzungen zusätzlich mindestens Protein bzw. Proteinhydrolysat, ausgewählt aus mindestens einem Vertreter der Gruppe, die gebildet wird aus Erbsenprotein, Sojaprotein, Mandelprotein, Akazienprotein, Collagen und Keratin sowie deren Hydrolysate, enthalten.

Es ist erfindungsgemäß bevorzugt, wenn die erfindungsgemäßen Zusammensetzungen zusätzlich mindestens ein kationisches Polymer und/oder mindestens ein amphoteres Polmer enthalten.

Unter kationischen Polymeren sind Polymere zu verstehen, welche in der Haupt-und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind; haben sich als besonders geeignet erwiesen.

Unter dem Begriff amphotere Polymere werden solche Polymere verstanden,
- die im Molekül sowohl freie Aminogruppen als auch freie -COOH- oder SO₃H-Gruppen enthalten und zur Ausbildung innerer Salze befähigt sind,
- zwitterionische Polymere, die im Molekül quartäre Ammoniumgruppen und -COO-Gruppen oder -SO₃⁻-Gruppen enthalten, sowie
- Polymere, die -COOH-Gruppen oder SO₃H-Gruppen und quartäre Ammoniumgruppen enthalten.
Die in der Aufzählung genannten Polymere mit quartären Ammoniumgruppen werden erfindungsgemäß bevorzugt als amphotere Polymere eingesetzt.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Homopolymere von Diallylammoniumsalzen, Copolymere von Diallylammoniumsalzen und Acrylamiden, quaternierte Vinyl-pyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Beispiele für solche kationischen Polymere sind:
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen.

Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure.. Bevorzugt werden niedermolekulare Hydroxycarbonsäuren wie z.B. Glycolsäure oder Milchsäure verwendet.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere und/oder kationische Polymere sind solche Polymerisate, in denen sich eine kationische Gruppe ableitet von mindestens einem der folgenden Monomere:
(M1) Monomeren mit quartären Ammoniumgruppen der allgemeinen Formel (IV),

   R¹-CH=CR²-CO-Z-(CₙH₂ₙ)-N⁽⁺⁾R³R⁴R⁵ A⁻ (IV)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist,
(M2) Monomeren mit quartären Ammoniumgruppen der allgemeinen Formel (V), 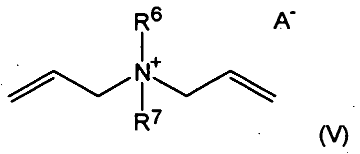
   worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist.

Dabei kann es sich um Homopolymere oder Copolymere handeln, die die Monomere (M1) und/oder (M2) enthalten.

Wenn sich eine kationische Gruppe der amphoteren bzw. kationischen Polymerisate vom Monomer des Typs (M1) ableitet, stehen in Formel (IV) die Reste R³, R⁴ und R⁵ bevorzugt für Methylgruppen, Z ist bevorzugt eine NH-Gruppe und A⁻ bedeutet bevorzugt ein Halogenid-, Methoxysulfat- oder Ethoxysulfat-Ion. Besonders bevorzugt ist es in diesem Falle Acrylamidopropyl-trimethyl-ammoniumchlorid als Monomer (M1) zu verwenden.

In Formel (V) steht A⁻ bevorzugt für ein Halogenidion, insbesondere für Chlorid oder Bromid.

Ein besonders geeignetes Homopolymer aus Monomer (M1) ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Kationische Copolymere mit Monomereinheiten (M1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Das bevorzugte kationische -Polymer aus dem Monomer mit der Formel (V) ist das entsprechende Homopolymer. Solche Homopolymere werden beispielsweise als Diallyldimethylammonium-Homopolymere mit der INCI-Bezeichnung Polyquaternium-6 zum Beispiel unter dem Handelsnamen Merquat^{®} 100 (Nalco) vermarktet.

Bevorzugte erfindungsgemäße amphotere Polymere sind Polymere, deren anionische Gruppe sich von mindestens einem Monomeren der Formel (VI) ableitet
(M3) monomeren Carbonsäuren der allgemeinen Formel (VI) bzw. deren Salze mit einer organischen oder anorganischen Säure,

   R⁸-CH=CR⁸-COOH (VI)

   in denen R⁸ und R⁹ unabhängig voneinander Wasserstoff oder Methylgruppen sind.

Die amphoteren Polymere können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

Als Monomeres (M3) wird für die erfindungsgemäß bevorzugten amphoteren Polymerisate Acrylsäure verwendet.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben.

Die amphoteren bzw. kationischen Polymere sind in dem erfindungsgemäßen Mittel bevorzugt in einer Menge von 0,05 bis 15 Gew.%, besonders bevorzugt von 0,1 bis 5 Gew.-% jeweils bezogen auf das Gewicht des anwendungsbereiten Mittels enthalten.

Die erfindungsgemäßen Zusammensetzungen können ebenso als gelförmige Reiniger für Oberflächen, wie z.B. als Toilettenreiniger, verwendet werden. Die besagten gelförmigen Zusammensetzungen eignen sich besonders zur Ausbringung aus einem Behältnis mit Pumpsystem.

Ein zweiter Gegenstand der Erfindung ist ein oxidatives Haarfärbemittel. Dieses wird unmittelbar vor der Anwendung durch Mischung der Zubereitung des Oxidationsmittels mit der gelförmigen Zusammensetzung des ersten Erfindungsgegenstandes, enthaltend mindestens ein Oxidationsfarbstoffvorprodukt in Form von Entwickler- und/oder Kupplerkomponenten, im Gewichtsverhältnis von 1:2 bis 2:1 hergestellt. Das dabei entstehende gebrauchsfertige Haarfärbepräparat ist ebenso gelförmig und sollte bevorzugt einen pH-Wert im Bereich von pH 6 bis 12, insbesondere von pH 7,5 bis 11, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt bevorzugt, da die Zusammensetzung des ersten Erfindungsgegenstandes tensidhaltig ist.

Die Oxidationsmittelzubereitung enthält besonders bevorzugt

| | |
|---|---|
| 1 bis 24 Gew.-% | Wasserstoffperoxid |
| | |
| 0,1 bis 5 Gew.-% | eines wasserlöslichen; synthetischen Tensids und |
| | |
| 1 bis 5 Gew.-% | eines dispergierten Acrylsäure- und/oder Methacrylsäure-Polymerisats oder -Copolymerisats |

im wäßrigen Träger neben den in solchen Zubereitungen üblichen Stabilisierungshilfsmitteln. Sie kann aber auch im einfachsten Fall allein aus Wasser bestehen, so daß die Oxidation durch den Luftsauerstoff herbeigeführt wird. Bevorzugt wiederum wird in obiger Oxidationsmittelzubereitung das Wasserstoffperoxid in einer Menge von 1 bis 12 Gew.-% eingesetzt.

Als wasserlösliche synthetische Tenside können in der Oxidationsmittelzubereitung die bereits für die als Trägermedium fungierende erfindungsgemäße Zusammensetzung genannten anionischen, amphoteren, zwitterionischen und nichtionischen Tenside oder Gemische davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt.

Die Oxidationsmittelzubereitung enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit des alkalischen erfindungsgemäßen Trägermediums, das Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich im wäßrigen Medium zu lösen und die Viskosität der Lösung anzuheben.

Besonders günstig für den Viskositätsaufbau nach dem Vermischen der erfindungsgemäßen Zusammensetzung und der Oxidationsmittelzubereitung ist der Gehalt an dispergierten Acrylsäure- und/oder Methacrylsäure-Polymerisat oder - Copolymerisat in der Oxidationsmittelzubereitung. Solche Dispersionen von Copolymerisaten, z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren, sind z.B. in GB 870 994 beschrieben. Aus DE 11 64 095 sind Mischpolymerisate aus 50 bis 75 Gew.-% Ethylacrylat, 25 bis 35 Gew.-% Acrylsäure und 0 bis 25 Gew.-% anderer Comonomerer bekannt. Geeignete Dispersionen dieser Art sind im Handel erhältlich, z.B. unter der Handelsbezeichnung Latekoll^{®} D (BASF). Besonders gut eignen sich die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Die erfindungsgemäßen Haarfärbemittel können zusätzlich alle auf diesen Gebieten bekannten und üblicherweise verwendeten Wirk- und Hilfsstoffe enthalten.

Die erfindungsgemäßen Haarfärbemittel weisen bevorzugt einen pH-Wert von 5 bis 11, insbesondere von 8 bis 10, auf.

Ein dritter Gegenstand ist die Verwendung einer gelförmigen Zusammensetzung des ersten Erfindungsgegenstandes als Trägermedium für Oxidationsfarbstoffvorprodukte und/oder direktziehende Farbstoffe.

Für den dritten Erfindungsgegenstand gilt mutatis mutandis das für den ersten und zweiten Erfindungsgegenstand Gesagte.

Ein vierter Erfindungsgegenstand ist die Verwendung einer gelförmigen Zusammensetzung des ersten Erfindungsgegenstandes zur Applikation auf Oberflächen, wie beispielsweise zur Reinigung einer Oberfläche, insbesondere Haut, Haare oder harte Oberflächen (z.B. im Haushalt wie Böden, Arbeitsflächen, Mobiliar). Hier kommt das erfindungsgemäße Mittel als Träger eines Waschgels, Shampoos bzw. Oberflächenreinigers zum Einsatz.

Die folgenden Beispiele sollen den Gegenstand der Erfindung näher erläutern:

### Beispiele

Es wurden folgende gelförmige Zusammensetzungen gemäß Tabelle 1 als kosmetische Träger für Oxidationsfarbstoffvorprodukte bereitgestellt. Die Mengenangaben sind Gewichtsprozent bezogen auf das Gewicht des gesamten Mittels.

**Tabelle 1: Gelförmiqe Zusammensetzung**

| Rohstoff | E1 | E2 | E3 | E4 | V1 |
|---|---|---|---|---|---|
| Dehydol^{®} LS 2 Deo N ¹ | 5,00 | 5,00 | 5,00 | 5,00 | 5,00 |
| Eumulgin^{®} CS 50 ⁵ | 1,00 | 1,00 | 1,00 | 1,00 | 1,00 |
| Kokoslorol^{®} C12-18 ² | 5,00 | 5,00 | 5,00 | 5,00 | 5,00 |
| Edenor^{®} PK 1805 ³ | 5,00 | 5,00 | 5,00 | 5,00 | 5,00 |
| 1,2-Propylenglykol | 2,00 | 2,00 | 2,00 | 2,00 | 2,00 |
| Texapon^{®} NSO UP ⁴ | 3,50 | 3,50 | 3,50 | 3,50 | 3,50 |
| 2-Aminoethanol | 6,50 | 6,50 | 6,50 | 6,50 | 6,50 |
| Parfumöl | 0,60 | 0,60 | 0,60 | 0,60 | 0,60 |
| L-Arginin | 1,00 | 1,00 | 1,00 | 1,00 | 1,00 |
| Ascorbinsäure | 0,20 | 0,20 | 0,20 | 0,20 | 0,20 |
| Natriumsulfit | 0,20 | 0,20 | 0,20 | 0,20 | 0,20 |
| Turpinal^{®} SL ⁶ | 0,20 | 0,20 | 0,20 | 0,20 | 0,20 |
| Isopropylmyristat | 3,00 | 5,00 | 7,00 | 10,00 | - |
| Natronwasserglas 40/42 | 0,50 | 0,50 | 0,50 | 0,50 | 0,50 |
| p-Toluylendiamin | 5,20 | 5,20 | 5,20 | 5,20 | 5,20 |
| 4-Amino-3-methylphenol | 0,12 | 0,12 | 0,12 | 0,12 | 0,12 |
| 1,5-Dihydroxynaphthalin | 0,04 | 0,04 | 0,04 | 0,04 | 0,04 |
| Resorcin | 0,41 | 0,41 | 0,41 | 0,41 | 0,41 |
| 4-Chlorresorcin | 0,47 | 0,47 | 0,47 | 0,47 | 0,47 |
| m-Aminophenol | 0,77 | 0,77 | 0,77 | 0,77 | 0,77 |
| 5-Amino-2-methylphenol | 0,05 | 0,05 | 0,05 | 0,05 | 0,05 |
| 2-Amino=3-methylamino-6-methoxypyridin | 0,56 | 0,56 | 0,56 | 0,56 | 0,56 |
| Wasser | add 100 | add 100 | add 100 | add 100 | add 100 |
| Viskosität geschert [mPa·s] | 32450 | 27000 | 15100 | 13000 | 36050 |
| Viskosität geruht [mPa·s] | 1650 | 2700 | 5100 | 7150 | 38700 |

| | | | | | |
|---|---|---|---|---|---|
| 1 C₁₂-C₁₄ Fettalkohol mit 2 Einheiten Ethylenoxid (INCI-Bezeichnung: Laureth-2) (Hersteller: COGNIS) 2 C₁₂-C₁₈-Fettalkohol (INCI-Bezeichnung: Coconut Alcohol) (Hersteller: COGNIS) 3 Kaliumoleat (Hersteller: COGNIS) 4 Natriumlaurylethersulfat (27 % Aktivsubstanz; INCI: Sodium Laureth Sulfate) (Hersteller : COGNIS) 5 C₁₆-C₁₈ Fettalkohol mit ca. 50 Einheiten Ethylenoxid (INCI-Bezeichnung: Ceteareth-50) (Hersteller: COGNIS) 6 1-Hydroxyethan-1,1-diphosphonsäure (INCI-Bezeichnung: Etidronic Acid, Aqua (Water)) (Hersteller: Solutia) | | | | | |

Die Viskositäten wurden jeweils mit dem Viskometer Brookfield DV II+ pro bei einer Temperatur von 22,5°C mit der Spindel Nr. 4 bei 4 Umdrehungen pro Minute gemessen. Zur Messung der Viskosität des ruhenden Gels (Viskosität geruht) wurde die gelförmige Zusammensetzung in ein Becherglas gefüllt und 30 Minuten ruhen gelassen. Dann wurde die Viskosität gemessen und der Messwert nach einer Messdauer von 1 Minute abgelesen. Zur Bestimmung der Viskosität eines gescherten Gels (Viskosität geschert) wurde die in das Becherglas gefüllte gelförmige Zusammensetzung für 15 Sekunden mit einem Glasstab stark gerührt. Dann wurde die Viskosität gemessen und der Messwert nach einer Messdauer von 1 Minute abgelesen. Die Viskosität der gescherten Zusammensetzungen E1 bis E4 bleibt nach dem Rühren für mehrere Stunden erhöht.

## Patentansprüche

1. Gelförmige Zusammensetzung, enthaltend
- Wasser,
- 1,0 bis 15 Gew.% mindestens eines gesättigten oder ungesättigten, linearen oder verzweigten Alkohols mit 8 bis 36 C-Atomen,
- 0,1 bis 15 Gew.-% mindestens einer flüssigen Fettsäure mit 16 bis 22 C-Atomen in Form einer wasserlöslichen Seife,
- 0,5 bis 10 Gew.-% mindestens eines Anlagerungsproduktes von 1 bis 5 Mol Ethylenoxid an einen linearen Fettalkohol mit 8 bis 22 C-Atomen,
**dadurch gekennzeichnet, daß** sie zusätzlich
- 0,5 bis 10 Gew.-% mindestens eines Esteröls ausgewählt aus
(i) C₁- bis C₁₀-Alkyl-(C₈- bis C₃₀-)alkanoaten, deren Kohlenwasserstoffreste linear oder verzweigt, gesättigt oder ungesättigt sein können und/oder
(ii) C₈- bis C₃₀-Alkyl-(C₁- bis C₁₀-)alkanoaten, deren Kohlenwasserstoffreste linear oder verzweigt, gesättigt oder ungesättigt sein können, enthält.

2. Gelförmige Zusammensetzung nach Anspruch 1, **dadurch gekennzeichnet, dass** sie als gesättigten oder ungesättigten, linearen oder verzweigten Alkohol mit 8 bis 36 C-Atomen Fettalkohole mit 12 bis 18 Kohlenstoffatomen enthält.

3. Gelförmige Zusammensetzung nach, einem der Ansprüche 1 oder 2, **dadurch gekennzeichnet, daß** sie zusätzlich 0,5 bis 10 Gew.-% mindestens eines Anlagerungsproduktes von 15 bis 100 Mol Ethylenoxid an einen linearen Fettalkohol mit 8 bis 22 C-Atomen enthält.

4. Gelförmige Zusammensetzung nach einem der Ansprüche 1 bis 3, **dadurch gekennzeichnet, dass** sie zusätzlich mindestens ein Polyol enthält.

5. Gelförmige Zusammensetzung nach einem der Ansprüche 1 bis 4, **dadurch gekennzeichnet, dass** sie zusätzlich ein wasserlösliches synthetisches Tensid, ausgewählt aus der Gruppe der kationischen, amphoteren, zwitterionischen Tenside und der Fettalkylglycoside, enthält.

6. Gelförmige Zusammensetzung nach einem der Ansprüche 1 bis 5, **dadurch gekennzeichnet, dass** sie zusätzlich Dicetylstearylether, Dicetylstearyloxiethylether, Dicetylstearyldioxiethylether und/oder N,N-Bis(2-Cetylstearyloxiethyl)-aminoethanol enthält.

7. Gelförmige Zusammensetzung nach einem der Ansprüche 1 bis 6, **dadurch gekennzeichnet, dass** das Esteröl in einer Menge von 1 bis 5 Gew.-% enthalten ist.

8. Gelförmige Zusammensetzung nach einem der Ansprüche 1 bis 7, **dadurch gekennzeichnet, dass** das Esteröl aus mindestens einer Verbindung gemäß Formel (II) ausgewählt wird 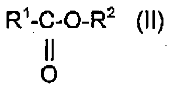 worin
einer der Reste R¹ oder R² für einen linearen oder verzweigten, gesättigten oder ungesättigten (C₂- bis C₁₀)-Kohlenwasserstoffrest steht und der jeweils andere Rest einen linearen oder verzweigten, gesättigten oder ungesättigten (C₈- bis C₂₂)-Kohlenwasserstoffrest bedeutet.

9. Gelförmige Zusammensetzung nach einem der Ansprüche 1 bis 8, **dadurch gekennzeichnet, dass** sie zusätzlich ein Protein bzw. Proteinhydrolysat, ausgewählt aus mindestens einem Vertreter der Gruppe, die gebildet wird aus Erbsenprotein, Sojaprotein, Mandelprotein, Akazienprotein, Collagen und Keratin sowie deren Hydrolysate, enthält.

10. Gelförmige Zusammensetzung nach einem der Ansprüche 1 bis 9, **dadurch gekennzeichnet, dass** sie zusätzlich mindestens ein kationisches Polymer und/oder mindestens ein amphoteres Polymer enthält.

11. Gelförmige Zusammensetzung nach einem der Ansprüche 1 bis 10, **dadurch gekennzeichnet, dass** sie zusätzlich mindestens ein Oxidationsfarbstoffvorprodukt vom Entwicklertyp und/oder Kupplertyp enthält.

12. Haarfärbemittel, welches unmittelbar vor dem Aufbringen auf das Haar aus einem Trägermedium, enthaltend mindestens ein Oxidationsfarbstoffvorprodukt vom Entwicklertyp und/oder Kupplertyp, und einer fließfähigen, wäßrigen Oxidationsmittelzubereitung, miteinander im Gewichtsverhältnis 1:2 bis 2:1 zu einem gelförmigen Färbeansatz vermischt werden, **dadurch gekennzeichnet, daß** das Trägermedium eine gelförmige Zusammensetzung nach einem der Ansprüche 1 bis 11 ist.

13. Haarfärbemittel nach Anspruch 12, **dadurch gekennzeichnet, dass** die Oxidationsmittelzubereitung
- 1 bis 24 Gew.% Wasserstoffperoxid,
- 0,1 bis 5 Gew.-% eines wasserlöslichen synthetischen Tensids und
- 1 bis 5 Gew.-% eines dispergierten Acrylsäure und/oder Methacrylsäure-polymerisats oder -Copolymerisats
enthält.

14. Verwendung einer gelförmigen Zusammensetzung nach einem der Ansprüche 1 bis 11 als Trägermedium für Oxidationsfarbstoffvorprodukte und/oder direktziehende Farbstoffe.

15. Verwendung einer gelförmigen Zusammensetzung nach einem der Ansprüche 1 bis 11 zur Applikation auf Oberflächen, insbesondere Haut, Haare oder harte Oberflächen.

## Claims

1. A gel-like composition, containing:
- water,
- 1.0 to 15% by weight of at least one saturated or unsaturated, linear or branched alcohol with 8 to 36 C atoms,
- 0.1 to 15% by weight of at least one liquid fatty acid with 16 to 22 C atoms in the form of a water-soluble soap,
- 0.5 to 10% by weight of at least one addition product of 1 to 5 moles of ethylene oxide onto a linear fatty alcohol with 8 to 22 C atoms, **characterized in that** it further comprises
- 0.5 to 10% by weight of at least one ester oil selected from
(i) (C₁-C₁₀)alkyl-(C₈-C₃₀)alkanoates, the hydrocarbon radicals of which may be saturated or unsaturated, linear or branched
and/or
(ii) (C₈-C₃₀)alkyl-(C₁-C₁₀)alkanoates, the hydrocarbon radicals of which may be saturated or unsaturated, linear or branched.

2. The gel-like composition according to claim 1, **characterized in that** it contains as a saturated or unsaturated, linear or branched alcohol with 8 to 36 C atoms, fatty alcohols with 12 to 18 carbon atoms.

3. The gel-like composition according to one of claims 1 or 2, **characterized in that** it further contains 0.5 to 10% by weight of at least one addition product of 15 to 100 moles of ethylene oxide on a linear fatty alcohol with 8 to 22 C atoms.

4. The gel-like composition according to one of claims 1 to 3, **characterized in that** it further contains at least one polyol.

5. The gel-like composition according to one of claims 1 to 4, **characterized in that** it further contains a water-soluble synthetic surfactant, selected from the group of cationic, amphoteric, zwitterionic surfactants and fatty alkylglycosides.

6. The gel-like composition according to one of claims 1 to 5, **characterized in that** it further contains dicetylstearylether, dicetylstearyloxyethylether, dicetylstearyldioxyethylether and/or N, N-bis(2-cetylstearyloxyethyl)aminoethanol.

7. The gel-like composition according to one of claims 1 to 6, **characterized in that** the ester oil is contained in an amount from 1 to 5% by weight.

8. The gel-like composition according to one of claims 1 to 7, **characterized in that** the ester oil is selected from at least one compound according to formula (II) 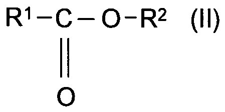 wherein
one of the radicals R¹ or R² stands for a saturated or unsaturated, linear or branched C₂-C₁₀ hydrocarbon radical and respectively the other radical represents a saturated or unsaturated, linear or branched C₈-C₂₂ hydrocarbon radical.

9. The gel-like composition according to one of claims 1 to 8, **characterized in that** it further contains a protein, respectively a protein hydrolyzate, selected from at least one representative of the group which is formed with pea protein, soya bean protein, almond protein, acacia protein, collagen and keratin as well as hydrolyzates thereof.

10. The gel-like composition according to one of claims 1 to 9, **characterized in that** it further contains at least one cationic polymer and/or at least one amphoteric polymer.

11. The gel-like composition according to one of claims 1 to 10, **characterized in that** it further contains at least one oxidation dye precursor product of the developer type and/or a coupler type.

12. A hair dye, which, consisting of a carrier medium containing at least one oxidation dye precursor product of the developer type and/or a coupler type, and of a flowable aqueous oxidation agent preparation, is mixed together in a weight proportion from 1:2 to 2:1, into a gel-like dye mixture directly before application on the hair, **characterized in that** the carrier medium is a gel-like composition according to one of claims 1 to 11.

13. The hair dye according to claim 12, **characterized in that** the oxidation agent preparation contains:
- 1 to 24% by weight of hydrogen peroxide,
- 0.1 to 5% by weight of a water-soluble synthetic surfactant and
- 1 to 5% by weight of a dispersed acrylic acid and/or methacrylic acid polymer or copolymer.

14. The use of a gel-like composition according to one of claims 1 to 11, as a carrier medium for oxidation dye precursor products and/or substantive dyes.

15. The use of a gel-like composition according to one of claims 1 to 11, for application on surfaces, in particular the skin, hair or hard surfaces.

## Revendications

1. Composition sous forme de gel, contenant
- de l'eau ;
- de 1,0 à 15 % en poids d'au moins un alcool saturé ou insaturé, linéaire ou ramifié contenant de 8 à 36 atomes de carbone ;
- de 0,1 à 15 % en poids d'au moins un acide gras liquide contenant de 16 à 22 atomes de carbone sous la forme d'un savon soluble dans l'eau ;
- de 0,5 à 10 % en poids d'au moins un produit de fixation par addition de 1 à 5 mol d'oxyde d'éthylène à un alcool gras linéaire contenant de 8 à 22 atomes de carbone ;
**caractérisée en ce qu'**elle contient en outre
- de 0,5 à 10 % en poids d'au moins une huile d'ester choisie parmi
(i) des alkyl(en C₁-C₁₀)alcanoates en C₈-C₃₀ dont les résidus d'hydrocarbures peuvent être linéaires ou ramifiés, saturés ou insaturés ;
et/ou
(ii) des alkyl(en C₈-C₃₀)alcanoates en C₁-C₁₀ dont les résidus d'hydrocarbures peuvent être linéaires ou ramifiés, saturés ou insaturés.

2. Composition sous forme de gel selon la revendication 1, **caractérisée en ce qu'**elle contient comme alcool saturé ou insaturé, linéaire ou ramifié contenant de 8 à 36 atomes de carbone, des alcools gras contenant de 12 à 18 atomes de carbone.

3. Composition sous forme de gel selon l'une quelconque des revendications 1 ou 2, **caractérisée en ce qu'**elle contient en outre de 0,5 à 10 % en poids d'au moins un produit de fixation par addition de 15 à 100 mol d'oxyde d'éthylène à un alcool gras linéaire contenant de 8 à 22 atomes de carbone.

4. Composition sous forme de gel selon l'une quelconque des revendications 1 à 3, **caractérisée en ce qu'**elle contient en outre au moins un polyol.

5. Composition sous forme de gel selon l'une quelconque des revendications 1 à 4, **caractérisée en ce qu'**elle contient en outre un agent tensioactif synthétique soluble dans l'eau choisi parmi le groupe des agents tensioactifs cationiques, amphotères, zwitterioniques et des glycosides d'alkyle gras.

6. Composition sous forme de gel selon l'une quelconque des revendications 1 à 5, **caractérisée en ce qu'**elle contient en outre de l'éther dicétylstéarylique, de l'éther dicétylstéaryloxyéthylique, de l'éther dicétylstéaryldioxyéthylique et/ou le N,N-bis(2-cétylstéaryloxyéthyl)-aminoéthanol.

7. Composition sous forme de gel selon l'une quelconque des revendications 1 à 6, **caractérisée en ce qu'**elle contient l'huile d'ester en une quantité de 1 à 5 % en poids.

8. Composition sous forme de gel selon l'une quelconque des revendications 1 à 7, **caractérisée en ce que** l'huile d'ester est choisie parmi au moins un composé répondant à la formule (II) 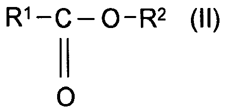 dans laquelle
un des résidus R¹ ou R² représente un résidu d'hydrocarbure en C₂-C₁₀ linéaire ou ramifié, saturé ou insaturé et respectivement l'autre le résidu représente un résidu d'hydrocarbure en C₈-C₂₂ linéaire ou ramifié, saturé ou insaturé.

9. Composition sous forme de gel selon l'une quelconque des revendications 1 à 8, **caractérisée en ce qu'**elle contient en outre une protéine respectivement un hydrolysat de protéine choisi parmi au moins un représentant du groupe qui est formé par de la protéine de petit pois, de la protéine de soja, de la protéine d'amande, de la protéine de gomme d'acacia, du collagène et de la kératine, ainsi que leurs hydrolysats.

10. Composition sous forme de gel selon l'une quelconque des revendications 1 à 9, **caractérisée en ce qu'**elle contient en outre au moins un polymère cationique et/ou au moins un polymère amphotère.

11. Composition sous forme de gel selon l'une quelconque des revendications 1 à 10, **caractérisée en ce qu'**elle contient en outre au moins un précurseur de colorant d'oxydation de type développeur et/ou de type coupleur.

12. Agent de teinture capillaire que l'on mélange directement avant l'application sur les cheveux à partir d'un milieu de support, contenant au moins un précurseur de colorant d'oxydation de type développeur et/ou de type coupleur, et à partir d'une préparation aqueuse fluide d'agent d'oxydation, l'un avec l'autre dans le rapport pondéral 1:2 à 2:1 pour obtenir une préparation de teinture sous forme de gel, **caractérisé en ce que** le milieu de support représente une composition sous forme de gel selon l'une quelconque des revendications 1 à 11.

13. Agent de teinture capillaire selon la revendication 12, **caractérisé en ce que** la préparation d'agent d'oxydation contient
- de 1 à 24 % en poids de peroxyde d'hydrogène ;
- de 0,1 à 5 % en poids d'un agent tensioactif synthétique soluble dans l'eau ; et
- de 1 à 5 % en poids d'un polymère ou d'un copolymère d'acide acrylique et/ou d'acide méthacrylique à l'état dispersé.

14. Utilisation d'une composition sous forme de gel selon l'une quelconque des revendications 1 à 11, à titre de milieu de support pour des précurseurs de colorants d'oxydation et/ou pour des colorants directs.

15. Utilisation d'une composition sous forme de gel selon l'une quelconque des revendications 1 à 11, pour l'application sur des surfaces, en particulier la peau, les cheveux ou des surfaces dures.
